# EUROPÄISCHE PATENTSCHRIFT

(11) **EP 0 800 392 B1**
(45) Veröffentlichungstag und Bekanntmachung des Hinweises auf die Patenterteilung: **06.02.2002**
(21) Anmeldenummer: 95905606.0
(22) Anmeldetag: 29.12.1994
(51) Int. Cl.: A61K 31/44, A61K 9/14

(54) **PHARMAZEUTISCHE ZUBEREITUNGEN, ENTHALTEND NIFEDIPIN UND VERFAHREN ZU IHRER HERSTELLUNG**
PHARMACEUTICAL PREPARATIONS CONTAINING NIFEDIPINE AND PROCESS FOR PRODUCING IT
PREPARATIONS PHARMACEUTIQUES CONTENANT DE LA NIFEDIPINE ET PROCEDES PERMETTANT DE LES PREPARER

(43) Veröffentlichungstag der Anmeldung: 15.10.1997
(73) Patentinhaber: Shire Deutschland GmbH & Co. KG., 50679 Köln (DE)
(72) Erfinder: WALCH, Hatto, D-88471 Laupheim (DE); PETSZULAT, Monika, D-88471 Laupheim (DE); MUSKULUS, Frank, D-88471 Laupheim (DE)
(74) Vertreter: Sandmair, Kurt, Dr. Dr.
(86) Internationale Anmeldenummer: EP9404334
(87) Internationale Veröffentlichungsnummer: WO9620707

(56) Entgegenhaltungen:
- EP-A- 0 047 899
- EP-A- 0 385 582
- WO-A-86/01717
- PATENT ABSTRACTS OF JAPAN vol. 18 no. 251 (C-1199) ,13.Mai 1994 & JP,A,06 032737 (NIPPON IYAKUHIN KOGYO KK) 8.Februar 1994,

## Beschreibung

Die vorliegende Erfindung betrifft neue pharmazeutische Zubereitungen für die orale Verabreichung mit einem Gehalt an 1,4-Dihydro-2,6-dimethyl-4-(o-Nitrophenyl)-3,5-pyridin-dicarbonsäure-dimethylester (Nifedipin) als Wirkstoff, wie sie in den Ansprüchen im einzelnen gekennzeichnet und im folgenden näher erläutert sind.

Der Wirkstoff Nifedipin gehört zur Gruppe der Calciumantagonisten und wird aufgrund seiner koronargefäßerweiternden und hypotensiven Wirkung zur Behandlung der koronaren Herzkrankheit und des Bluthochdruckes eingesetzt.

Aufgrund der schlechten Löslichkeit des Nifedipins treten bei der galenischen Verarbeitung eine Reihe von Schwierigkeiten auf, wie aus zahlreichen Publikationen und Patentanmeldungen über spezielle Formulierungen mit Nifedipin ersichtlich wird.

Bislang vorgeschlagene pharmazeutische Zubereitungen mit Nifedipin basieren auf der Verwendung von oberflächenaktiven Stoffen und/oder wasserlöslichen Trägerstoffen. So beschreibt die DE-A-2 822 882 feste, glasartige Copräzipitate, die ein Gemisch aus Nifedipin und mindestens einem Hilfsstoff, wie Polyvinylpyrrolidon oder einem Cellulosederivat darstellen. In der DE-A-3 413 634 werden Mischungen von Nifedipin und Zuckeralkoholen beansprucht. Die DE-A-3 438 830 beschreibt eine Darreichungsform, in der das Nifedipin molekulardispers in einer erstarrten Schmelze, die eine Mischung aus flüssigen und festen Polyethylenglykolen darstellt, vorliegt.

Zur Therapie von Bluthochdruck und koronaren Herzerkrankungen benötigt man Arzneizubereitungen, die nicht nur zu rasch ansteigenden Plasmaspiegeln führen, sondern auch die notwendigen Wirkstoffkonzentrationen im Plasma innerhalb der therapeutisch empfohlenen Dosierungsintervalle aufrechterhalten. Da das Nifedipin eine relativ kurze Eliminationshalbwertszeit besitzt, lassen sich diese Voraussetzungen nur schwer erfüllen. Mit üblichen, schnell freisetzenden Arzneiformen lassen sich die notwendigen Nifedipin-Plasmaspiegel nicht über den geforderten Zeitraum aufrechterhalten, so daß eine zweckmäßige Therapie des Bluthochdrucks und der koronaren Herzerkrankungen nicht durchgeführt werden kann.

Inzwischen sind verschiedene Lösungswege zur Herstellung von Darreichungsformen mit langanhaltenden Blutspiegeln beschrieben worden. Die EP-A-0 047 899 beschreibt Arzneizubereitungen, die nicht nur zu einem raschen Anstieg des Nifedipin-Spiegels führen, sondern auch die Plasmakonzentrationen über viele Stunden auf einem hohen Wert halten sollen. Dem Prinzip liegt zugrunde, Nifedipin-Kristalle unterschiedlicher Größe (Nifedipin-Kristalle mit einer spezifischen Oberfläche von 1 bis 4 m²/g) zu kombinieren. Um Produkte mit einer großen spezifischen Oberfläche von bis zu 4 m²/g zu erhalten, sind aufwendige Mahlverfahren, wie z.B. die Mikronisierung durch eine Luftstrahlmühle, durchzuführen. (Siehe auch das Patent EP-B-047 899).

Das in dieser Schrift beschriebene Verfahren hat außerdem den Nachteil, daß bei Mahlungen mit Stift- oder Hammermühlen die Nifedipin-Kristalle nicht in der gewünschten, breiten Korngrößenverteilung anfallen. So läßt sich z.B. mit einer Stiftmühle auch bei niedriger Drehzahl nur eine relativ schmale Korngrößenverteilung erzielen, wobei das Material oft zu mehr als 95 % kleiner als 50 µm anfällt. Um das in der genannten Patentpublikation geforderte Material zu erhalten, ist es notwendig, das Nifedipin-Mahlgut zunächst auszusieben und anschließend entsprechende Mengen innerhalb der geforderten Kornfraktion zu vermischen. Dieses Verfahren ist umso aufwendiger, als die gesamten Herstellungsschritte aufgrund der hohen Lichtinstabilität der Substanz unter Lichtschutz durchgeführt werden müssen. Auch ein Vermischen von Fraktionen außerhalb der bezeichneten spezifischen Oberfläche mit dem Ziel, rein rechnerisch den gewünschten Bereich einzustellen, führt nicht zu dem gewünschten Ergebnis eines lang anhaltenden, ausreichenden hohen Plasmaspiegels an Nifedipin.

Die DE-A-3 636 123 schlägt Zubereitungssysteme vor, die neben einem Dihydropyridin einen Fettalkohol oder ein Fettalkoholgemisch in Kombination mit Polyethylenglykolen mittleren Molekulargewichts enthalten, wodurch eine plateauartige Freisetzung des Wirkstoffes über 24 Stunden erzielt wird. Darüberhinaus ist mit dem Einsatz an zusätzlichen Hilfsstoffen in Form von Retardiermitteln ein erhöhter Aufwand bei einem derartigen Herstellungsverfahren verbunden und führt zu großdimensionierten Darreichungsformen.

Generell ist festzuhalten, daß alle Versuche, die schlechte Löslichkeit von Nifedipin durch bestimmte Maßnahmen zu kompensieren und gleichzeitig eine gute Bioverfügbarkeit zu gewährleisten, eine Reihe von Nachteilen aufweisen. Der Einsatz von oberflächenaktiven Substanzen, Lösungsvermittlern und bestimmten Trägerstoffen, die eine besondere Oberfläche haben, führt häufig zu Darreichungsformen, bei dem die Präparate, bzw. einzunehmenden Dosiseinheiten unerwünscht groß sind. Außerdem sind dafür umständliche und kostenintensive Herstellungsverfahren notwendig.

Der Erfindung liegt somit die Aufgabe zugrunde, eine Retardform von Nifedipin zu schaffen, welche diese Nachteile nicht aufweist. Die erfindungsgemäße Aufgabe besteht darin, im Hinblick auf den bisherigen Stand der Technik retardierte, Nifedipin enthaltende Arzneizubereitungen zu schaffen, welche im Vergleich zu dem bisherigen Stand der Technik mindestens vergleichbare, lang anhaltende, hohe oder verbesserte Plasmakonzentrationen an Nifedipin ermöglichen, wobei sich diese Retardformen technisch in einfacher Weise herstellen lassen sollten und deren Wirkung trotz des Weglassens zusätzlicher Retardierhilfsmittel klinisch reproduzierbare Ergebnisse zeitigt.

Als Lösung dieser Aufgabe wurde nun überraschend gefunden, daß es möglich ist, unter Verwendung von Nifedipin einer spezifischen Oberfläche in dem sehr schmalen Fraktionsbereich von 0,2 bis 0,5 m²/g, vorzugsweise von 0,3 bis 0,4 m²/g pharmazeutische Zubereitungen herzustellen, die hinsichtlich der Bioverfügbarkeit des Wirkstoffes den im Handel befindlichen Präparationen mindestens ebenbürtig oder sogar überlegen sind, obwohl entgegen des Erfordernisses gemäß der EP-A-0 047 899 eine spezifische Oberfläche in einem völlig anderen und obendrein äußerst schmalen Bereich der spezifischen Oberfläche liegt und der Zusatz an zum Teil vielfältigen retardierenden Hilfsstoffen gemäß zahlreichen Versuchen des Standes der Technik nicht benötigt wird.

Die erfindungsgemäßen Retard-Arzneimittel sind bei vergleichbar guter Bioverfügbarkeit wirtschaftlicher herzustellen, da komplizierte, kostenintensive Verfahrensschritte ausgeschaltet sind. Die Erfindung betrifft demnach Retardarzneimittel, die Nifedipin als Wirkstoff enthalten und wie folgt zusammengesetzt sind:
- a): Nifedipin als Wirkstoff mit einer spezifischen Oberfläche von 0,2 bis 0,5 m²/g, vorzugsweise von 0,3 bis 0,4 m²/g sowie
- b): übliche pharmazeutisch verwendbare Hilfsstoffe.

Die erfindungsgemäß verwendeten Nifedipin-Kristalle mit einer spezifischen Oberfläche von 0,2 bis 0,5 m²/g, vorzugsweise von 0,3 bis 0,4 m²/g werden durch die Steuerung der Kristallisation erhalten. Die synthesemäßig anfallenden Kristalle liegen bereits in einem sehr engen Korngrößenbereich in dem entsprechenden Bereich der spezifischen Oberfläche vor und werden anschließend gesiebt, um eventuell auftretende Agglomerate zu zerteilen, bzw. geringe Mengen außerhalb des gewünschten Bereichs liegende Fraktionen zu entfernen. Somit ist das äußerst aufwendige, kostenintensive Verfahren der Mikronisierung, beispielsweise mittels einer Luftstrahlmühle, mit denen Produkte mit Werten einer größeren spezifischen Oberflächen erhalten werden, vermieden.

Die spezifische Oberfläche wird nach der Gasadsorptionsmethode (BET-Methode) bestimmt.

Zur Herstellung der erfindungsgemäßen Zubereitungen wird Nifedipin der genannten spezifischen Oberfläche mit üblichen pharmazeutischen Hilfsstoffen in einfacher Weise vermischt und zu den gewünschten, insbesondere zu festen, peroral applizierbaren Darreichungsformen verarbeitet. Besonders geeignet und kostengünstig ist dabei das Abfüllen der pulverförmigen Wirkstoff-/Hilfsstoffmischung in Hartgelatinekapseln.

Die so hergestellten Zubereitungen zeigen in überraschender Weise eine unerwartet hohe Bioverfügbarkeit. Es konnte nicht erwartet werden, daß nach peroraler Applikation der erfindungsgemäßen Zubereitungen mit kristallinem Nifedipin, welches eine wesentlich kleinere spezifische Oberfläche als das in der EP-B-47 899 beschriebene Nifedipin aufweist, die Plasmakonzentration schnell ansteigt und sich für viele Stunden auf einem hohen Niveau hält.

Das folgende Beispiel dient der Erläuterung der Erfindung.

### Beispiel 1:

| | |
|---|---|
| Nifedipin (spezif. Oberfläche 0,2 bis 0,5 m²/g, | 20,00 mg |
| vorzugsweise 0,3 bis 0,4 m²/g) Laktose | 153,50 mg |
| Hochdisperses Siliciumdioxid | 1,00 mg |
| Silikonisiertes Talkum | 4,00 mg |
| Magnesiumstearat | 1,50 mg |
| Gesamtmenge | 180,00 mg |

Die genannten Bestandteile werden miteinander vermischt und als Pulver in Hartgelatine-Kapseln abgefüllt. Es lassen sich in analoger Weise auch Weichgelatine-Kapseln herstellen, wobei ggf. als Träger für Weichgelatine-Kapseln übliche Hilfsstoffe, in denen Nifedipin unlöslich ist, in Betracht kommen.

Es kommen dabei auch Überzüge aus üblichen Hilfsstoffen, beispielsweise aus Gründen des Lichtschutzes, zur gezielten Freisetzung an bestimmten Orten des Gastrointestinaltraktes, des ästhetischen Aussehens, Geschmackes oder einer erleichterten Einnahme in Betracht.

Die Hilfs-, Träger- und Zusatzstoffe lassen sich dabei jeweils in gewünschter Weise variieren, ebenso wie die jeweiligen Wirkstoffdosen.

Es lassen sich auch Suppositorien zur rektalen Applikation oder Suspensionen zur peroralen Verabreichung mit jeweils langanhaltender Wirkung herstellen.

## Patentansprüche

1. Retardarzneimittel mit einem Gehalt an Nifedipin als Wirkstoff, **dadurch gekennzeichnet, daß** das Nifedipin in Form von Kristallen mit einer spezifischen Oberfläche von 0,2 bis 0,5 m²/g in Abwesenheit von retardierenden Hilfsstoffen vorliegt.

2. Retardarzneimittel gemäß Anspruch 1, **dadurch gekennzeichnet, daß** das Nifedipin mit einer spezifischen Oberfläche von 0,3 bis 0,4 m²/g vorliegt.

3. Retardarzneimittel gemäß den Ansprüchen 1 und 2, **dadurch gekennzeichnet, daß** das Retardarzneimittel in Form von Kapseln vorliegt.

4. Retardarzneimittel gemäß den Ansprüchen 1 bis 3, **dadurch gekennzeichnet, daß** es in Form von Hartgelatinekapseln vorliegt.

5. Retardarzneimittel gemäß den Ansprüchen 1 bis 3, **dadurch gekennzeichnet, daß** es in Form von Weichgelatinekapseln vorliegt.

6. Retardarzneimittel gemäß den Ansprüchen 1 bis 5, **dadurch gekennzeichnet, daß** der Trägerstoff aus Laktose besteht.

7. Verwendung von Nifedipin mit einer spezifischen Oberfläche von 0,2 bis 0,5 m²/g zur Herstellung von Retardarzneimitteln ohne den Zusatz an retardierenden Hilfsstoffen.

8. Verwendung von Nifedipin mit einer spezifischen Oberfläche von 0,3 bis 0,4 m²/g gemäß Anspruch 7.

9. Verwendung von Nifedipin gemäß den Ansprüchen 7 und 8 zur Herstellung von Retardkapselpräparaten.

10. Verwendung von Nifedipin gemäß Anspruch 9 zur Herstellung von Hartgelatineretardkapseln.

11. Verwendung von Nifedipin gemäß Anspruch 9 zur Herstellung von Weichgelatineretardkapseln.

12. Verwendung von Nifedipin gemäß den Ansprüchen 7 und 8 zur Herstellung von Suppositorien mit lang anhaltender Wirkung.

13. Verfahren zur Herstellung von Nifedipin enthaltenden Retardarzneimitteln gemäß den Ansprüchen 1 und 2, **dadurch gekennzeichnet, daß** der Wirkstoff Nifedipin in Form von feinen Kristallen mit einer spezifischen Oberfläche von 0,2 bis 0,5 m²/g, vorzugsweise von 0,3 bis 0,4 m²/g zu der gewünschten Retardarzneiform verarbeitet wird.

14. Verfahren gemäß Anspruch 13, **dadurch gekennzeichnet, daß** das Nifedipin mit einer spezifischen Oberfläche von 0,3 bis 0,4 m²/g zusammen mit Laktose als Trägerstoff und mit kleinen Anteilen an konventionellen Trenn-, Gleit- und Gegenklebemitteln, jedoch ohne den Zusatz an retardierenden Hilfsstoffen als Pulver vermischt und anschließend dieses Pulvergemisch in Hartgelatinekapseln zur Herstellung eines Retardpräparates abgefüllt wird.

15. Verfahren gemäß Anspruch 13, **dadurch gekennzeichnet, daß** das Nifedipin mit einer spezifischen Oberfläche von 0,2 bis 0,5 m²/g, vorzugsweise von 0,3 bis 0,4 m²/g zu Weichgelatineretardkapseln ohne den Zusatz an Retardierhilfsmitteln verarbeitet wird.

## Claims

1. Retard pharmaceutical preparation containing Nifedipin as active ingredient, **characterized in that** the Nifedipin is present in the form of crystals with a specific surface area of 0.2 to 0.5 m²/g in the absence of retarding adjuvants.

2. Retard pharmaceutical preparation according to Claim 1, **characterized in that** the Nifedipin is present with a specific surface area of 0.3 to 0.4 m²/g.

3. Retard pharmaceutical preparation according to Claims 1 and 2, **characterized in that** the retard pharmaceutical preparation is present in the form of capsules.

4. Retard pharmaceutical preparation according to Claims 1 to 3, **characterized in that** it is present in the form of hard gelatine capsules.

5. Retard pharmaceutical preparation according to Claims 1 to 3, **characterized in that** it is present in the form of soft gelatine capsules.

6. Retard pharmaceutical preparation according to Claims 1 to 5, **characterized in that** the support material consists of lactose.

7. Use of Nifedipin with a specific surface area of 0.2 to 0.5 m²/g to manufacture retard pharmaceutical preparations without the addition of retarding adjuvants.

8. Use of Nifedipin with a specific surface area of 0.3 to 0.4 m²/g according to Claim 7.

9. Use of Nifedipin according to Claims 7 and 8 to manufacture retard capsule preparations.

10. Use of Nifedipin according to Claim 9 to manufacture hard gelatine retard capsules.

11. Use of Nifedipin according to Claim 9 to manufacture soft gelatine retard capsules.

12. Use of Nifedipin according to Claims 7 and 8 to manufacture suppositories with an activity that is sustained for a long time.

13. Process for the manufacture of retard pharmaceutical preparations containing Nifedipin according to Claims 1 and 2, **characterized in that** the active ingredient Nifedipin in the form of fine crystals with a specific surface area of 0.2 to 0.5 m²/g, preferably of 0.3 to 0.4 m²/g, is processed to yield the required retard pharmaceutical form.

14. Process according to Claim 13, **characterized in that** Nifedipin with a specific surface area of 0.3 to 0.4 m²/g is blended as a powder together with lactose as carrier substance and with small proportions of conventional release, lubricant and anti-cake agents but without the addition of retarding adjuvants, and afterwards this powder mixture is packed into hard gelatine capsules to manufacture a retard preparation.

15. Process according to Claim 13, **characterized in that** the Nifedipin with a specific surface area of 0.2 to 0.5 m²/g, preferably of 0.3 to 0.4 m²/g, is processed to yield soft gelatine retard capsules without the addition of retarding adjuvants.

## Revendications

1. Médicament à effet retard ayant une teneur en nifédipine comme substance active, **caractérisé en ce que** la nifédipine est présente sous forme de cristaux avec une surface spécifique de 0,2 à 0,5 m²/g en l'absence de substances auxiliaires retardatrices.

2. Médicament à effet retard selon la revendication 1, **caractérisé en ce que** la nifédipine est présente avec une surface spécifique de 0,3 à 0,4 m²/g.

3. Médicament à effet retard selon les revendications 1 et 2, **caractérisé en ce que** le médicament retard est sous la forme de capsules.

4. Médicament à effet retard selon les revendications 1 à 3, **caractérisé en ce qu'**il est sous la forme de capsules de gélatine dure.

5. Médicament à effet retard selon les revendications 1 à 3, **caractérisé en ce qu'**il est sous la forme de capsules de gélatine molle.

6. Médicament à effet retard selon les revendications 1 à 5, **caractérisé en ce** le matériau de support est constitué de lactose.

7. Utilisation de nifédipine ayant une surface spécifique de 0,2 à 0,5 m²/g pour la fabrication de médicaments à effet retard sans addition de substances auxiliaires retardatrices.

8. Utilisation selon la revendication 7 de nifédipine ayant une surface spécifique de 0,3 à 0,4 m²/g.

9. Utilisation de nifédipine selon les revendications 7 et 8 pour la fabrication de préparations de capsules à effet retard.

10. Utilisation de nifédipine selon la revendication 9 pour la fabrication de capsules à effet retard en gélatine dure.

11. Utilisation de nifédipine selon la revendication 9 pour la fabrication de capsules à effet retard en gélatine molle.

12. Utilisation de nifédipine selon les revendications 7 et 8 pour la fabrication de suppositoires à effet longuement persistant.

13. Procédé pour la fabrication de médicaments à effet retard contenant de la nifédipine selon les revendications 1 et 2, **caractérisé en ce que** la nifédipine est mise en oeuvre sous la forme de fins cristaux ayant une surface spécifique de 0,2 à 0,5 m²/g, de préférence de 0,3 à 0,4 m²/g pour obtenir la forme retard souhaitée du médicament.

14. Procédé selon la revendication 13, **caractérisé en ce que** de la nifédipine ayant une surface spécifique de 0,3 à 0,4 m²/g est mélangée à l'état de poudre avec du lactose comme support et avec de faibles proportions d'agent séparateur, d'agent lubrifiant, et d'agent anti-adhérent, cependant sans addition de substances auxiliaires retardatrices, et ensuite ce mélange de poudres est introduit dans des capsules en gélatine dure pour la fabrication d'une préparation à effet retard.

15. Procédé selon la revendication 13, **caractérisé en ce que** la nifédipine ayant une surface spécifique de 0,2 à 0,5 m²/g, de préférence de 0,3 à 0,4 m²/g est mise en oeuvre pour obtenir des capsules à effet retard en gélatine molle sans addition de substances auxiliaires retardatrices.
